(19)

Europäisches
Patentamt
European
Patent Office
Office européen
des brevets

(11) **EP 4 207 212 A1**

(12) **EUROPEAN PATENT APPLICATION**

(43) Date of publication:
**05.07.2023 Bulletin 2023/27**

(21) Application number: **22214919.7**

(22) Date of filing: **20.12.2022**

(51) International Patent Classification (IPC):
**G16H 20/30** (2018.01)      **A63B 24/00** (2006.01)

(52) Cooperative Patent Classification (CPC):
**G16H 20/30; A63B 21/0628; A63B 21/4047;**
**A63B 23/03525; A63B 23/1209; A63B 71/0622;**
A63B 21/4035; A63B 2071/0658; A63B 2208/0233;
A63B 2220/13; A63B 2220/805; A63B 2220/806;
A63B 2225/15; A63B 2225/50

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **28.12.2021 KR 20210190376**
**14.11.2022 KR 20220152001**

(71) Applicant: **DRAX Inc.**
**Anyang-si, Gyeonggi-do 14086 (KR)**

(72) Inventor: **YOO, Seon Kyung**
**Seoul (KR)**

(74) Representative: **Mewburn Ellis LLP**
**Aurora Building**
**Counterslip**
**Bristol BS1 6BX (GB)**

(54) **ARTIFICIAL INTELLIGENCE WORKOUT GUIDE APPARATUS AND METHOD**

(57)      Provided is an artificial intelligence (AI) workout guide apparatus including a personal maximum weight (PMW) estimator configured to estimate $PMW_{estimation}$ of fitness equipment to be used by a user, based on an estimated muscular strength value calculated based on user data, and a PMW guider configured to provide $PMW_{individual}$ for the fitness equipment by complementing the $PMW_{estimation}$ with an individual objectification index of the user

EP 4 207 212 A1

**Description**

CROSS-REFERENCE TO RELATED APPLICATIONS

This application is based on and claims priority under 35 U.S.C. §119 to Korean Patent Application Nos. 10-2021-0190376, filed on December 28, 2021 and 10-2022-0152001, filed on November 14, 2022, in the Korean Intellectual Property Office, the disclosures of which are incorporated by reference herein in their entireties.

BACKGROUND

1. Field

The disclosure relates to a method of automatically providing a target weight of fitness equipment, based on the individual characteristics of a user.

2. Description of the Related Art

Weight fitness equipment is provided in various forms according to a purpose of use or a body part for increasing muscular strength, and is configured to train an upper body and a lower body by mainly using hands or feet. A user may work out by moving a selected weight through a fitness structure of fitness equipment.

However, it is difficult for the user to determine whether he/she is appropriately using the fitness equipment according to his/her physical characteristics, workout capability, or a purpose of the fitness equipment.

SUMMARY

Provided is an artificial intelligence (AI) workout guide apparatus for providing an appropriate initial target weight of fitness equipment used by a user by using only user data including the user's gender, age, weight, height, body mass index (BMI), and body fat percentage.

Provided is an AI workout guide apparatus for automatically adjusting a target weight of fitness equipment according to a purpose of workout of a user.

Provided is a method of newly setting and providing appropriate target weights to a first user and a second user, who have a same gender, age, weight, height, BMI, and body fat percentage, for each piece of fitness equipment used by the first user and the second user by further reflecting individual objectification indexes including athletic performances of the first user and the second user, even when a same initial target weight is provided to the first user and the second user for each piece of fitness equipment.

Provided is a method by which an AI workout guide apparatus learns and analyzes individual objectification indexes of users and continuously updates a target weight of fitness equipment differentiated for each user, thereby enabling the user to use the fitness equipment in an optimum manner.

Additional aspects will be set forth in part in the description which follows and, in part, will be apparent from the description, or may be learned by practice of the presented embodiments of the disclosure.

According to an aspect of the disclosure, an artificial intelligence (AI) workout guide apparatus includes a personal maximum weight (PMW) estimator configured to estimate $PMW_{estimation}$ of fitness equipment to be used by a user, based on an estimated muscular strength value calculated based on user data, and a PMW guider configured to provide $PMW_{individual}$ for the fitness equipment by complementing the $PMW_{estimation}$ with an individual objectification index of the user wherein the individual objectification index includes at least some of a weight of the fitness equipment, a number of repetitions (reps), a number of sets, a workout trajectory, a moving velocity, and regularity of reps in units of sets, which are determined when the user uses the fitness equipment for a pre-set period of time.

The AI workout guide apparatus may further include a workout target setter configured to automatically set an initial target weight of the fitness equipment to be used by the user, based on the $PMW_{estimation}$.

The AI workout guide apparatus may further include an AI workout target setter configured to automatically update a target weight of the fitness equipment to be used by the user, based on the $PMW_{individual}$.

The PMW guider may include a PMW updater configured to update the $PMW_{individual}$ to a value greater than the $PMW_{estimation}$ when the individual objectification index is equal to or greater than a first reference value, and update the $PMW_{individual}$ to a value less than $PMW_{estimation}$ when the individual objectification index is equal to or less than a second reference value. The PMW updater may be further configured to newly update the updated $PMW_{individual}$ based on the individual objectification index in units of the pre-set periods of time.

The PMW guider may include a display configured to display a target weight of the fitness equipment, the target weight being automatically updated based on the $PMW_{individual}$. The display may be further configured to display a change amount of the target weight of the fitness equipment for the pre-set period of time.

The PMW guider may be further configured to, when the workout trajectory is used as the individual objectification index, determine completion of the workout trajectory by using an ascent starting point, a descent starting point, an ascending section average velocity, a descending section average velocity, and a height, which are determined from the workout trajectory, convert a degree of the completion of the workout trajectory into a numerical value, and use the numerical value as the individual objectification index.

The PMW guider may be further configured to, when the reps are used as the individual objectification index, determine completion of the reps based on regularity between workout trajectories of all reps configuring one set, convert a degree of the completion of the reps into a numerical value, and use the numerical value as the individual objectification index.

According to another aspect of the disclosure, an artificial intelligence (AI) workout guide method includes estimating, by a PMW estimator, $PMW_{estimation}$ of fitness equipment to be used by a user, based on an estimated muscular strength value calculated based on user data, and providing, by a PMW guider, $PMW_{individual}$ for the fitness equipment by complementing the $PMW_{estimation}$ with an individual objectification index of the user wherein the individual objectification index includes at least some of a weight of the fitness equipment, a number of repetitions (reps), a number of sets, a workout trajectory, a moving velocity, and regularity of reps in units of sets, which are determined when the user uses the fitness equipment for a pre-set period of time.

BRIEF DESCRIPTION OF THE DRAWINGS

The above and other aspects, features, and advantages of certain embodiments of the disclosure will be more apparent from the following description taken in conjunction with the accompanying drawings, in which:

FIG. 1 illustrates a smart gym system using an artificial intelligence (AI) workout guide apparatus, according to an embodiment;

FIG. 2 illustrates an example of fitness equipment provided in a smart gym and implementing an AI workout guide method, according to an embodiment;

FIG. 3 is a block diagram of an internal configuration of a smart gym server and fitness equipment implementing an AI workout guide method in a smart gym system, according to an embodiment;

FIG. 4 is a block diagram of an internal configuration of an AI workout guide apparatus, according to an embodiment;

FIG. 5 illustrates an example of a user interface of an AI workout guide apparatus, according to an embodiment;

FIG. 6 illustrates an example of a workout trajectory detected by fitness equipment implementing an AI workout guide method, according to an embodiment;

FIGS. 7 through 9 illustrate examples of an objectification index calculator analyzing based on the number of repetitions, according to an embodiment;

FIG. 10 illustrates an example of a machine learning processor classifying workout trajectory big data, according to an embodiment;

FIG. 11 illustrates an example of measuring workout trajectories of two users regarding a chest press machine, according to an embodiment; and

FIG. 12 is a flowchart of an AI workout guide method according to an embodiment.

DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. Expressions such as "at least one of," when preceding a list of elements, modify the entire list of elements and do not modify the individual elements of the list.

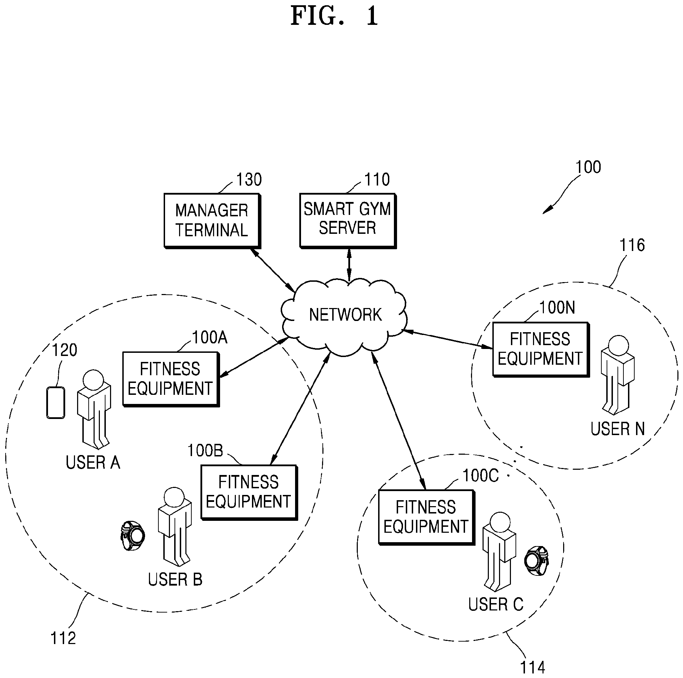

FIG. 1 illustrates a smart gym system 100 using an artificial intelligence (AI) workout guide apparatus, according to an embodiment.

The smart gym system 100 includes a smart gym server 110, at least one of pieces of fitness equipment 100a through 100n, at least one user terminal 120, and a manager terminal 130.

In FIG. 1, the smart gym server 110 may communicate with a first smart gym 112, a second smart gym 114, and an nth smart gym 116, which are physically at different locations, and may transmit or receive data to or from at least one of the pieces of fitness equipment 100a and 100b provided in the first smart gym 112, at least one piece of the fitness equipment 100c provided in the second smart gym 114, and at least one piece of the fitness equipment 100n provided in the nth smart gym 116.

According to an embodiment, a smart gym denotes a physical space that enables a user to provide an athletic performance using fitness equipment to the smart gym server 110, and the smart gym server 110 to learn and analyze the athletic performance of the user and provide workout prescription suitable to the user. The smart gym may be realized as a fitness center, a gym, or a space including fitness equipment.

Users USER A through USER N who come to the smart gym to exercise may enter the smart gym after identification when entering the smart gym. For example, the user may enter the smart gym through member verification by tagging the user terminal 120 to an unmanned terminal, such as a kiosk, at an entrance of the smart gym via near field communication (NFC) or radio frequency identification (RFID), or enter the smart gym after member verification at the unmanned terminal through bio-information identification, such as face recognition.

Information about the user who has been verified as a member may be transmitted from the smart gym server 110 to at least one of the pieces of fitness equipment 100a through 100n through a network. For example, the smart gym server 110 may transmit the information about the user to fitness equipment to which the user tagged the user terminal 120. According to an embodiment, the information about the user is also referred to as user data, and may include at least some or all of a gender, age, weight, height, body mass index (BMI), and body fat percentage.

The smart gym server 110 may guide the users USER A and USER B who use the fitness equipment 100a and 100b provided in the first smart gym 112, respectively, with a workout method, a workout intensity, and a workout sequence appropriate to each user. Also, the smart gym server 110 may provide values, such as a target weight and a recommended usage velocity, of the fitness equipment 100a and 100b. Also, the smart gym server 110 may receive athletic performances of the users USER A and USER B using the fitness equipment 100a and 100b, respectively. The smart gym server 110 may further receive, from the user terminal 120, log information or health information, such as a user's heart rate, blood pressure, and pulse.

The smart gym server 110 may be realized in a form of a cloud server. The smart gym server 110 may combine pieces of information collected from pieces of fitness equipment in smart gym fitness centers at different locations, and manage the pieces of information. For example, the smart gym server 110 may combine a history of a first user using fitness equipment in the first smart gym 112 at a first location and a history of the first user using fitness equipment in the second smart gym 114 at a second location, and manage the histories.

According to an embodiment, the at least one of the pieces of fitness equipment 100a through 100n may be stretching fitness equipment, weight fitness equipment, or aerobic fitness equipment. The weight fitness equipment includes free-weight equipment and machine equipment. The at least one of the pieces of fitness equipment 100a through 100n may provide an appropriate workout guide to a user through a display attached thereto or a display capable of communicating therewith via wires or wirelessly. For example, the stretching fitness equipment provides a workout guide related to stretching to be performed by a user, through a smart mirror capable of communicating with the stretching fitness equipment. However, an embodiment is not limited thereto, and a workout guide may be provided by using any one of various output methods, such as a speaker or vibration.

At least one of the pieces of fitness equipment 100a through 100n may perform wired/wireless communication with the smart gym server 110, the user terminal 120, and the manager terminal 130.

According to an embodiment, the user terminal 120 may be realized in a form of a smart phone, a smart watch, a handheld device, or a wearable device. Also, an application for using the smart gym system may be installed in the user terminal 120. The user terminal 120 may receive workout sequence information from the smart gym server 110. A workout sequence denotes a workout plan planned considering strength and workout capability of a user. The workout sequence includes information about a fitness equipment list to be used by the user, a target weight of each piece of fitness equipment, and the number of times each piece of fitness equipment is used.

When using at least one of the pieces of fitness equipment 100a through 100n in the smart gym system 100, a user may perform communication by tagging the user terminal 120 through NFC or RFID, or perform identification by using a physical characteristic of the user. When the identification of the user is completed, the smart gym server 110 may transmit user data to fitness equipment tagged by the user.

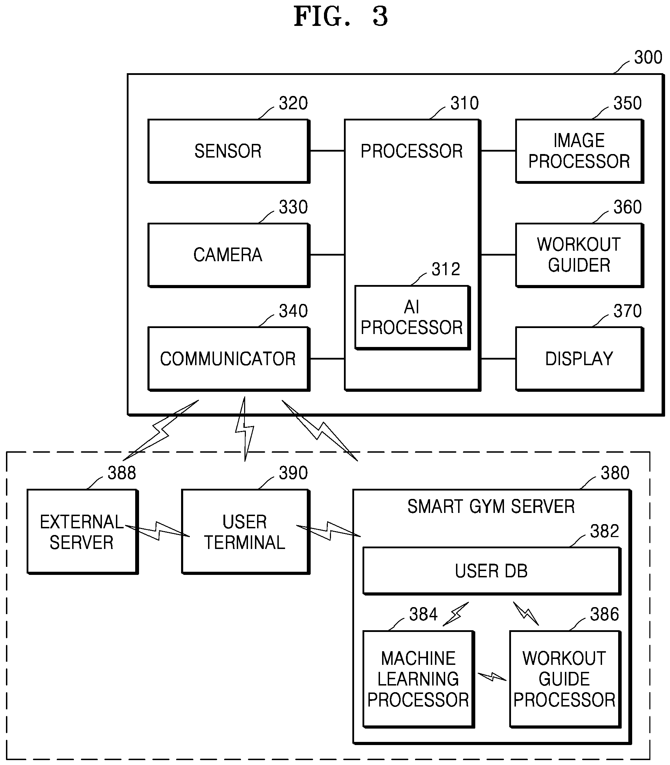

FIG. 3 is a block diagram of an internal configuration of a smart gym server 380 and fitness equipment 300 implementing an AI workout guide method in a smart gym system, according to an embodiment.

According to an embodiment, the fitness equipment 300 in a smart gym may communicate with the smart gym server 380, a user terminal 390, and an external server 388.

According to an embodiment, the fitness equipment 300 may include a processor 310, a sensor 320, a communicator 340, a workout guider 360, and a display 370. Also, the fitness equipment 300 may further include a camera 330 and an image processor 350. The processor 310 may include an AI processor 312 if necessary.

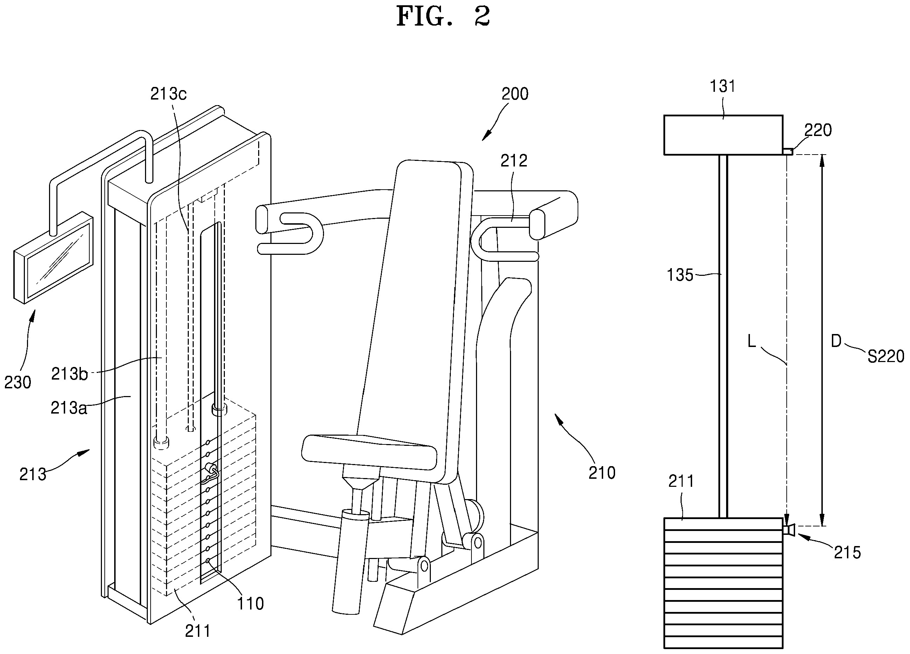

Further referring to FIG. 2, a shoulder press machine 200 is an example of the fitness equipment 300 to which the AI workout guide method used in the smart gym is applied. The fitness equipment 300 will be described with reference to FIGS. 2 and 3.

A sensor 220 may be installed at a frame structure 213 of a workout body 210. The frame structure 213 includes a base frame 213a, a guide rail 213b, and a connection line 213c. The sensor 220 irradiates a laser beam towards a pin structure 215, receives a reflected laser beam, and measures a distance D S220 from the sensor 220 to the pin structure 215 in real time or in units of pre-set t times. Accordingly, the sensor 220 may detect, in real time, at least one of a location, a moving velocity, and a moving direction of a weight member 211 selected by the pin structure 215. Also, when a user moves a weight plate by pushing a handle 212 of the shoulder press machine 200, the sensor 220 may measure the distance D S220 to the pin structure 215 embedded in the weight plate and detect a workout trajectory based on the distance D S220.

The communicator 340 may receive a user input through a display 230 or may transmit or receive user data to or from a user DB 382 of the smart gym server 380. The communicator 340 may also communicate with the external server 388.

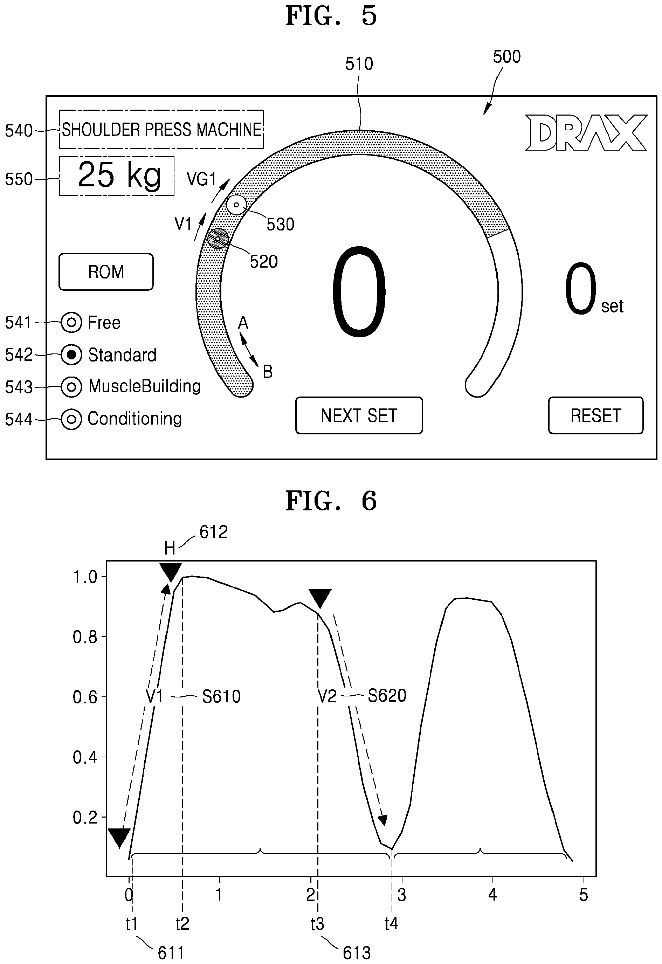

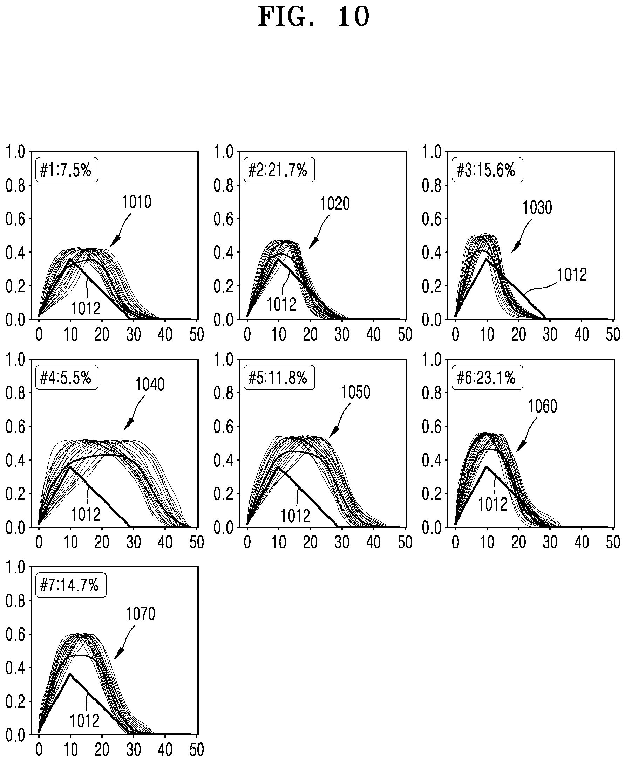

The workout guider 360 may provide, to the user, information received from the smart gym server 380, such as the user data, a target weight of fitness equipment, a moving velocity guide of the fitness equipment, a respiration guide when using the fitness equipment, and a workout sequence. An example by which the workout guider 360 provides the moving velocity guide of the fitness equipment to the user will be described below with reference to FIGS. 5 and 10.

Graphs of FIG. 10 show examples in which a machine learning processor 384 classified workout trajectories for specific fitness equipment obtained from a population into 7 patterns, i.e., first through seventh workout trajectories 1010 through 1070. The population initially aims at n pre-set people, but data of users using the smart gym may be continuously accumulated and used.

The machine learning processor 384 may classify the patterns of the workout trajectories of the population, and determine a proportion of the population belonging to each pattern. The machine learning processor 384 selects a workout trajectory having a highest workout effect from among the 7 patterns of workout trajectories. In this case, the machine learning processor 384 selects the second workout trajectory 1020 and the sixth workout trajectory 1060 that have a smallest deviation from a pre-set reference value trajectory 1012 having a highest workout effect. The second workout trajectory 1020 corresponds to 21.7 % of the population and the sixth workout trajectory 1060 corresponds to 23.1 % of the population.

A workout guide processor 386 may determine to which pattern a detected workout trajectory of a user belongs. Also, the workout guide processor 386 may determine a change in the workout trajectory of the user according to a workout load, the number of workouts, and workout hours. For example, in a case where a workout trajectory when a first user uses a chest press machine with 60 kg belongs to the second workout trajectory 1020, but a workout trajectory when the first user uses the chest press machine with 70 kg belongs to the first workout trajectory 1010, the workout guide processor 386 may determine that a load appropriate to the first user is 60 kg.

The workout guide processor 386 may provide, through the workout guider 360, a moving velocity guide VG1 530 of FIG. 5 of fitness equipment appropriate to a user. The moving velocity guide VG1 530 denotes a moving velocity appropriate to obtain an effect suitable to a purpose of workout when the user uses the fitness equipment. For example, when the user pushes the shoulder press machine 200 according to movement of the moving velocity guide VG1 530, a measured velocity V1 520 of the user pushing the shoulder press machine 200 is also displayed on a reference line 510.

The smart gym server 380 may calculate an estimated muscular strength value of specific muscular strength, based on user data. Then, the smart gym server 380 determines $PMW_{estimation}$ of fitness equipment to be used by the user, based on the estimated muscular strength value, and automatically sets an initial target weight of the fitness equipment to be used by the user, based on the $PMW_{estimation}$ and a purpose of workout of the user. According to an embodiment, a personal maximum weight (PMW) denotes muscular strength that may be exerted by an individual against a resistance of a weight with an utmost effort. Examples of the PMW include 1 RM and 4 RM. The $PMW_{estimation}$ indicates a value obtained by estimating a PMW of the user, based on the estimated muscular strength value, and the smart gym server 380 may determine $PMW_{estimation}$ per fitness equipment.

The smart gym server 380 may provide an initial target weight of the fitness equipment 300 to be used by the user to the workout guider 360 of the fitness equipment 300, and the workout guider 360 may display the initial target weight of the fitness equipment 300 to be used by the user on the display 370. FIG. 5 shows an example of suggesting an initial target weight 550 of FIG. 5 of fitness equipment to be used by a user.

The smart gym server 380 may also predict $PMW_{individual}$ that is a maximum muscular strength value obtained by complementing the $PMW_{estimation}$ with an individual objectification index of the user. In this case, the smart gym server 380 may provide a new target weight to which an individual characteristic of the user is additionally reflected, by updating the initial target weight of the fitness equipment 300 set based on the $PMW_{estimation}$. Examples of the individual objectification index include an athletic performance, a weight of fitness equipment 300 determined when the fitness equipment 300 is used, the number of repetitions (reps), the number of sets, a workout trajectory, a moving velocity, and regularity of reps in units of sets.

The camera 330 may be embedded in the fitness equipment 300 or may communicate with the fitness equipment 300 wirelessly or via wires, and may capture an image of a workout pose of the user. The image processor 350 may analyze the image of the workout pose of the user, which is captured by the camera 330, through the processor 310. Also, the image processor 350 may learn a result of processing the image of the workout pose, which is captured when the user works out according to a target weight of the fitness equipment 300, through the AI processor 312, and transmit a result of the learning to the smart gym server 380. The AI processor 312 may also process or learn the individual objectification index of the user.

The smart gym server 380 includes the user DB 382, the machine learning processor 384, and the workout guide processor 386. The user DB 382 stores and manages user data. The user data includes a gender, age, weight, height, BMI, and body fat percentage.

The machine learning processor 384 learns and processes the individual objectification index including athletic performances of the user on pieces of fitness equipment used in the smart gym. An example in which the machine learning processor 384 learns the individual objectification index will be described below with reference to FIGS. 6 through 11.

The machine learning processor 384 may update the $PMW_{estimation}$ to the $PMW_{individual,}$ based on the individual objectification indexes of the pieces of fitness equipment 300 used by the user in the smart gym for a certain period of time. The machine learning processor 384 may update the $PMW_{individual}$ to a value greater than $PMW_{estimation}$ when the individual objectification index is equal to or greater than a first reference value, and update the $PMW_{individual}$ to a value less than the $PMW_{estimation}$ when the individual objectification index is equal to or less than a second reference value.

When it is determined that workout capability of the user is greater than the $PMW_{estimation}$ measured from specific muscular strength of the user, based on the individual objectification index determined based on athletic performance of the user, the machine learning processor 384 may reset a maximum muscular strength value of the user to the $PMW_{individual}$ that is greater than the $PMW_{estimation}$. On the other hand, when it is determined that the workout capability of the user is less than the $PMW_{estimation}$ measured from the specific muscular strength of the user, based on the individual objectification index determined based on the athletic performance of the user, the machine learning processor 384 may reset the maximum muscular strength value of the user to the $PMW_{individual}$ that is less than the $PMW_{estimation}$.

Accordingly, the machine learning processor 384 may learn and predict $PMW_{individual}$ appropriate to a first user and a second user, who have a same gender, age, weight, height, BMI, and body fat percentage, for each piece of fitness equipment used by the first user and the second user by further reflecting individual objectification indexes including athletic performances of the first user and the second user, even when $PMW_{estimation}$ of the first user and the second user are estimated to be the same.

In other words, the machine learning processor 384 may learn and predict the $PMW_{individual}$ that is the maximum muscular strength value to which the individual objectification index varying according to a muscular development state, a movable range, body proportions, workout habits, and a workout achievement rate of each user is further reflected, even when user data related to physical conditions, such as a gender, age, weight, height, BMI, and body fat percentage, is the same.

The sensor 320 may also determine the workout trajectory, based on distance information sensed while the user uses the shoulder press machine 200. The machine learning processor 384 may determine completion of the workout trajectory by using at least some of an ascent starting point 611, a descent starting point 613, an ascending section velocity V1 S610, a descending section velocity V2 S620, an ascending section average velocity, a descending section average velocity, and a height H 612, which are determined from the workout trajectory detected while the user uses the shoulder press machine 200, convert a degree of the completion of the workout trajectory into a numerical value, and use the numerical value as the individual objectification index.

According to another embodiment, the machine learning processor 384 may, when reps is used as the individual objectification index, determine completion of the reps based on regularity between workout trajectories of all reps configuring one set, convert a degree of the completion of the reps into a numerical value, and use the numerical value.

The workout guide processor 386 may provide target weights of pieces of fitness equipment, updated based on the $PMW_{individual}$ received from the machine learning processor 384.

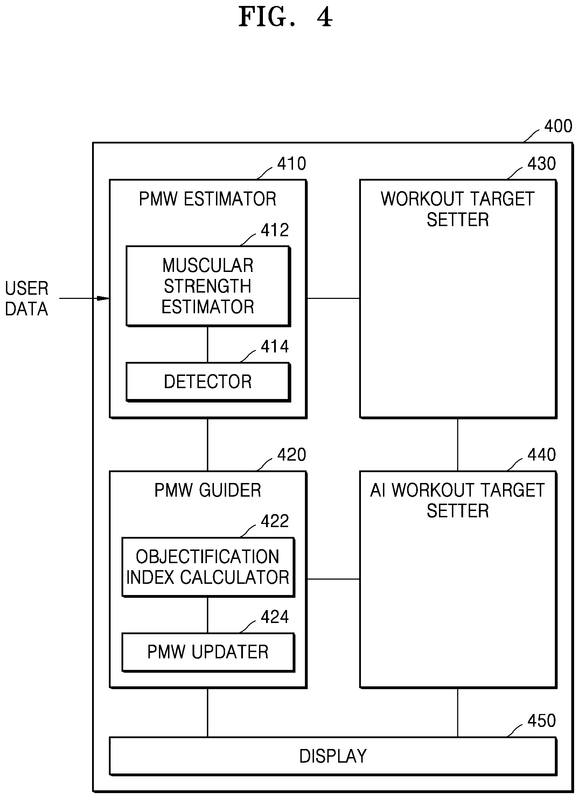

FIG. 4 is a block diagram of an internal configuration of an AI workout guide apparatus 400, according to an embodiment. The AI workout guide apparatus 400 may include a smart gym server or a terminal.

The AI workout guide apparatus 400 includes a PMW estimator 410, a PMW guider 420, a workout target setter 430, an AI workout target setter 440, and a display 450. The PMW estimator 410 includes a muscular strength estimator 412 and a detector 414. The PMW guider 420 includes an objectification index calculator 422 and a PMW updater 424.

The muscular strength estimator 412 estimates an estimated muscular strength value for specific muscular strength of a user, based on user data. The muscular strength estimator 412 calculates the estimated muscular strength value for the specific muscular strength of the user, as in Equation 1, by using information about the user's gender, BMI, body fat percentage, and age. A same estimated muscular strength value is calculated when the information about the user's gender, BMI, body fat percentage, and age is the same. An example of the specific muscular strength may include grasping power.

[Equation 1]

Estimated Muscular Strength Value =

$$A \times \text{Gender} + B \times \text{Age} + C \times \text{Body Fat Percentage} + D \times \text{BMI} + E$$

In Equation 1, A, B, C, D, and E denote pre-set values.

The detector 414 detects a percentile value to which the estimated muscular strength value belongs, from adult muscular strength percentile values obtained from the external server 388 of FIG. 3 or pre-set as in Table 1 or Table 2. Table 1 shows percentile of relative grasping power of adult men, and Table 2 shows percentile of relative grasping power of adult women.

[Table 1]

| Percentile | Age | | | | |
|---|---|---|---|---|---|
| | 19-29 | 30-39 | 40-49 | 50-59 | 60-69 |
| 90 | **.* | ... | | | **.* |
| ... | | | | | |
| 50 | 57.2 | | 56.1 | ** * | **.* |
| 40 | **.* | 55.9 | **.* | 53.8 | 49.2 |
| ... | | | | | |
| 10 | **.* | ... | | | **.* |

[Table 2]

| Percentile | Age | | | | |
|---|---|---|---|---|---|
| | 19-29 | 30-39 | 40-49 | 50-59 | 60-69 |
| 90 | **.* | ... | | | 47.5 |
| ... | | | | | |
| 50 | **.* | 46.3 | 43.8 | **.* | **.* |
| 40 | 40.3 | **.* | **.* | **.* | 34.1 |
| ... | | | | | |
| 10 | 31.7 | ... | | | **.* |

For example, when the muscular strength estimator 412 calculates an estimated muscular strength value of a 32-year-old man to be 55.9, the detector 414 detects, from adult muscular strength percentile values pre-set as in Table 1, a percentile value 40 to which the estimated muscular strength value 55.9 of the 32-year-old men belongs.

The PMW estimator 410 estimates $PMW_{estimation}$ of fitness equipment to be used by a user, by using a $PMW_{estimation}$ percentile value matched to the percentile value of the estimated muscular strength value detected by the detector 414. According to an embodiment, the PMW estimator 410 may download the $PMW_{estimation}$ percentile value from a smart gym server or pre-store the $PMW_{estimation}$ percentile value of fitness equipment, obtained from a population using the fitness equipment.

The smart gym server pre-stores a mapping table in which $PMW_{estimation}$ percentile values for pieces of fitness equipment are stored, and the $PMW_{estimation}$ percentile values may be updated in units of pre-set periods of time by further reflecting data of users using each piece of fitness equipment in the smart gym. In detail, the machine learning processor 384 of the smart gym server 380 generates the mapping table including the $PMW_{estimation}$ percentile values, based on PMW data obtained from an initially pre-set population. Then, the machine learning processor 384 may continuously collect the PMW data of the users using the pieces of fitness equipment provided in the smart gym, and update, in units of certain periods of time, the mapping table providing the $PMW_{estimation}$ percentile values, generated based on the PMW data obtained from the initially pre-set population.

Tables 3 through 5 show examples of mapping tables used by the PMW estimator 410. For convenience of descriptions, Tables 3 through 5 show only some of a PMW10 percentile value through a PMW90 percentile value, and it should be noted that the values are only examples for understanding the disclosure.

According to an embodiment, examples of the mapping table and PMW percentile values of fitness equipment obtained by the PMW estimator 410 from a population for a chest press machine are shown in Table 3.

[Table 3]

| PMW Percentile Value | Initial Population PMW (kg) | Updated Population PMW (kg) |
|---|---|---|
| PMW90 | 102.7 | 107.8 |
| ... | | |
| PMW50 | 67.2 | 67.8 |
| PMW40 | 65.1 | 64.7 |
| ... | | |
| PMW10 | 24.6 | 23.9 |

According to an embodiment, examples of the mapping table and PMW percentile values of fitness equipment obtained by the PMW estimator 410 from a population for a leg extension machine are shown in Table 4.

[Table 4]

| $PMW_{estimation}$ Percentile Value | Initial Population $PMW_{estimation}$ (kg) | Updated Population $PMW_{estimation}$ (kg) |
|---|---|---|
| PMW90 | 103.3 | 107.8 |
| ... | ... | ... |
| PMW50 | 67.3 | 69.2 |
| PMW40 | 66.5 | 66.8 |
| ... | ... | ... |
| PMW10 | 24.6 | 24.9 |

According to an embodiment, an example of the mapping table providing $PMW_{estimation}$ percentile values of fitness equipment obtained by the PMW estimator 410 from a population for a shoulder press machine is shown in Table 5.

[Table 5]

| PMW Percentile Value | Initial Population PMW (kg) | Updated Population PMW (kg) |
|---|---|---|
| PMW90 | 64.5 | 65.5 |
| ... | ... | ... |
| PMW50 | 43.3 | 43.2 |
| PMW40 | 41.2 | 40.2 |
| ... | ... | ... |
| PMW10 | 16.1 | 16.3 |

When a percentile value 40 to which an estimated muscular strength value 55.9 of a 32-year-old man belongs is detected from adult muscular strength percentile values pre-set as in Table 1, the PMW estimator 410 estimates a PMW of fitness equipment corresponding to the percentile value 40.

Referring to Tables 3 through 5, the PMW estimator 410 estimates $PMW_{estimation}$ of a chest press machine to be 65.1 kg corresponding to the percentile value of PMW40, based on "initial population PMW (kg)". The PMW estimator 410 estimates $PMW_{estimation}$ of a leg extension machine to be 66.5 kg corresponding to the percentile value of PMW40. Also, the PMW estimator 410 estimates $PMW_{estimation}$ of a shoulder press machine to be 41.2 kg corresponding to the percentile value of PMW40.

According to another embodiment, when big data of users using the fitness equipment in the smart gym is further collected in addition to the PMW data of the initially pre-set population, the PMW estimator 410 may use updated PMW data as in "updated population PMW (kg)" of Tables 3 through 5. In this case, the PMW estimator 410 may update the $PMW_{estimation}$ of the chest press machine to 64.7 kg corresponding to the percentile value of PMW40. The PMW estimator 410 may update the $PMW_{estimation}$ of the leg extension machine to 66.8 kg corresponding to the percentile value of PMW40. Also, the PMW estimator 410 may update the $PMW_{estimation}$ of the shoulder press machine to 40.2 kg corresponding to the percentile value of PMW40.

The workout target setter 430 sets an initial target weight of each piece of fitness equipment, based on $PMW_{estimation}$ of each piece of fitness equipment, estimated by the PMW estimator 410. The workout target setter 430 may set the initial target weight of each piece of fitness equipment by further reflecting a purpose of workout of a user, received through a user interface.

The workout target setter 430 may differently set the initial target weight of each piece of fitness equipment depending on whether the purpose of workout of the user is a free workout 541, a standard workout 542, a muscle building workout 543, or a conditioning workout 544.

In the free workout 541, the initial target weight of the fitness equipment may be set to $PMW_{estimation} \times P_{free}$ (%). In the standard workout 542, the initial target weight of the fitness equipment may be set to $PMW_{estimation} \times P_{standard}$ (%). In the muscle building workout 543, the initial target weight of the fitness equipment may be set to $PMW_{estimation} \times P_{muscle\ building}$ (%). Also, in the conditioning workout 544, the initial target weight of the fitness equipment may be set to $PMW_{estimation} \times P_{conditioning}$ (%).

According to an embodiment, the $P_{muscle\ building}$ (%) may be set to 65 to 85 %, and reps may be set to 6 to 12 times. According to an embodiment, when the purpose of workout is muscle building, the reflected weight $P_{muscle\ building}$ (%) may be a pre-set value. Also, the weight $P_{muscle\ building}$ (%) may be readjusted by receiving objectification index feedback, such as completion of a workout trajectory of the user regarding the initial target weight proposed to the user and a workout trajectory for each number of workouts of the user.

When the user has selected the standard workout 542 as the purpose of workout through a user interface provided on a display 500, the workout target setter 430 may reflect $P_{standard}$ (%) = 60 when the $PMW_{estimation}$ of the shoulder press machine is 40.2 kg, and then set 25 kg as the initial target weight for the user. 40.2 kg $\times$ 60 % = 24.12 kg, and 25 kg that is closest to a weight supported by the fitness equipment may be set as the initial target weight.

According to an embodiment, the PMW guider 420 provides $PMW_{individual}$ for the fitness equipment by complementing the $PMW_{estimation}$ estimated by the PMW estimator 410 with an individual objectification index of the user. The PMW guider 420 provides, to different users for whom a same $PMW_{estimation}$ has been estimated for same fitness equipment, $PMW_{individual}$ appropriate to each user by reflecting athletic performance of each user. Even when a same user uses a same piece of fitness equipment and a same $PMW_{estimation}$ has been estimated, the PMW guider 420 provides $PMW_{individual}$ appropriate to the user at a current time point by reflecting athletic performance of the user during a certain period of time. The PMW guider 420 may continuously provide a workout program appropriate to the user by using $PMW_{individual}$ to which a physical condition, physical strength, and a workout log of the user are reflected.

The PMW guider 420 may display, on the display 450, at least one of muscle activated when the user uses fitness equipment selected by the user, $PMW_{individual}$, and a target weight calculated based on the $PMW_{individual}$.

The PMW guider 420 includes the objectification index calculator 422 and the PMW updater 424. The objectification index calculator 422 calculates an individual objectification index by using at least some of a weight of the fitness equipment, reps, a number of sets, a workout trajectory, a moving velocity, and regularity of reps in units of sets, which are determined when the user uses the fitness equipment for a pre-set period of time.

The PMW updater 424 updates the $PMW_{individual}$ to a value greater than the $PMW_{estimation}$ when the individual objectification index calculated by the objectification index calculator 422 is equal to or greater than a first reference value, and updates the $PMW_{individual}$ to a value less than the $PMW_{estimation}$ when the individual objectification index is equal to or less than a second reference value. The PMW updater 424 may newly update the updated $PMW_{individual}$, based on the individual objectification index, in units of pre-set periods of time.

The AI workout target setter 440 updates a target weight of each piece of fitness equipment, based on the $PMW_{individual}$ calculated by the PMW guider 420. The AI workout target setter 440 may set the initial target weight of each piece of fitness equipment by further reflecting the purpose of workout of the user, received through the user interface. The target weight of each piece of fitness equipment is differently updated depending on whether the purpose of workout of the user is the free workout 541, the standard workout 542, the muscle building workout 543, or the conditioning workout 544.

According to an embodiment, a case where the objectification index calculator 422 uses the workout trajectory as the individual objectification index will be described with reference to FIG. 6. The objectification index calculator 422 determines the completion of the workout trajectory by using the ascent starting point 611, the descent starting point 613, the ascending section velocity V1 S610, the descending section velocity V2 S620, the ascending section average velocity, the descending section average velocity, and the height H 612, converts a degree of the completion of the workout trajectory into a numerical value, and uses the numerical value as the individual objectification index.

For example, the objectification index calculator 422 determines, according to a pre-set standard, that the ascent starting point 611 is appropriate when the ascent starting point 611 is 0 to $t_a$ seconds, and is late when the ascent starting point 611 exceeds $t_a$ seconds. Also, the completion may be rated good, fair, or bad or scored from 1 to 10, according to a pre-set standard for the ascent starting point 611. Similarly, according to a pre-set standard, when the ascending section velocity V1 S610 is between Va and Vb, it is determined that the ascending section velocity V1 S610 is appropriate, when the ascending section velocity V1 S610 exceeds Vb, it is determined that the ascending section velocity V1 S610 is fast, and when the ascending section velocity V1 S610 is less than Va, it is determined that the ascending section velocity V1 S610 is slow. A result of determining appropriate, fast, or slow may be rated good, fair, or bad or scored from 1 to 10.

Similarly, the objectification index calculator 422 determines the completion of the workout trajectory detected when the user uses the fitness equipment, by using at least some of the ascent starting point 611, the descent starting point 613, the ascending section velocity V1 S610, the descending section velocity V2 S620, the ascending section average velocity, the descending section average velocity, and the height H 612.

When the numerical value of the completion of the workout trajectory is used as the individual objectification index, the PMW updater 424 updates the $PMW_{individual}$ to a value greater than the $PMW_{estimation}$ when the individual objectification index is equal to or greater than the first reference value, for example, 8 points. For example, when the individual objectification index of the user is 9 points and the first reference value is 8 points, the $PMW_{individual}$ may be updated to a value greater than the $PMW_{estimation}$ by n % (n is a natural number). Also, when the individual objectification index is equal to or less than the second reference value, for example, 6 points, the $PMW_{individual}$ is updated to a value less than the $PMW_{estimation}$. Also, when the individual objectification index is greater than the second reference value and less than the first reference value, $PMW_{individual}$ is maintained to a value of the $PMW_{estimation}$.

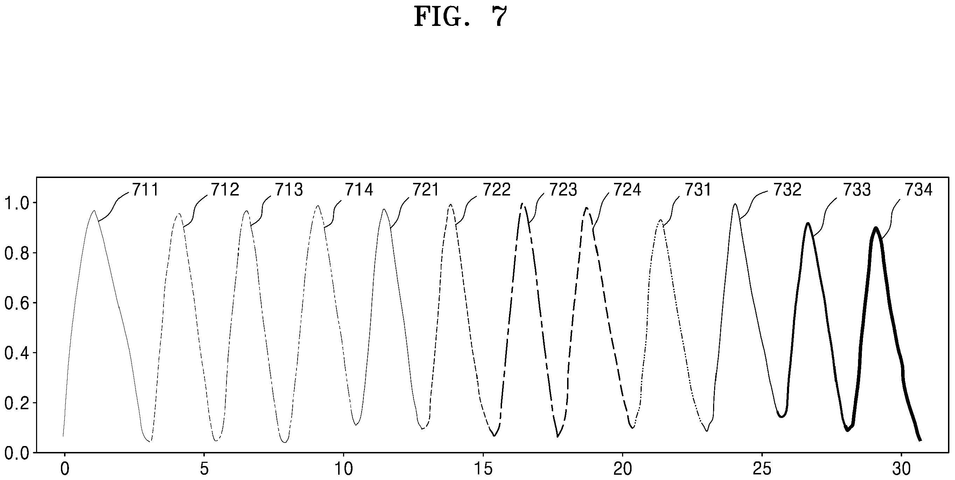

According to another embodiment, a case where the objectification index calculator 422 uses the reps as the individual objectification index will be described with reference to FIGS. 7 through 9. The objectification index calculator 422 determines completion of the reps, based on regularity between workout trajectories of all reps configuring one set, converts a degree of the completion of the reps into a numerical value, and uses the numerical value as the individual objectification index.

FIGS. 7 through 9 illustrate examples of workout trajectories detected while first through third users use seated leg press machines 12 times repeatedly during one set. In FIGS. 7 through 9, an x-axis denotes time and a y-axis denotes movement displacement of fitness equipment.

The objectification index calculator 422 determines whether reps in one set has been completed. The objectification index calculator 422 determines whether a user completed all 12 reps or quit and completed only some of the reps. Also, the objectification index calculator 422 determines the completion of the reps, based on the regularity between the workout trajectories of 12 reps 711 through 714, 721 through 724, and 731 through 734 of the first user, 12 reps 811 through 814, 821 through 824, and 831 through 834 of the second user, and 12 reps 911 through 914, 921 through 924, and 931 through 934 of the third user. In this case, the completion may be determined by dividing the regularity into early regularity, middle regularity, and latter regularity.

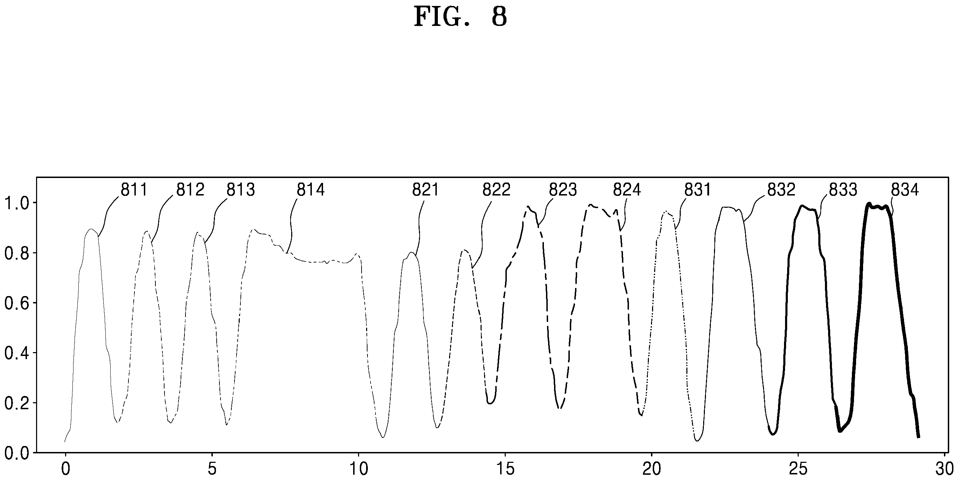

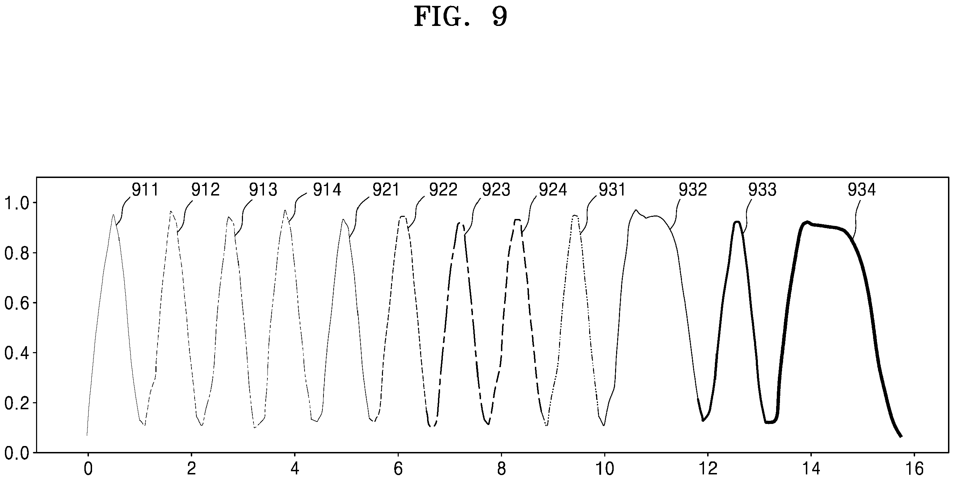

For example, the early regularity of the reps 711 through 714 of the first user of FIG. 7 and the reps 911 through 914 of the third user of FIG. 9 is high as the four workout trajectories are almost uniform. In this case, 4 points may be assigned to the early regularity. The workout trajectories of the reps 811 through 813 of the second user of FIG. 8 are uniform, but the workout trajectory of the rep 814 is deviated, and thus it is difficult to determine that the early regularity is high. In this case, 3 points may be assigned to the early regularity. However, this is only an example of assigning a numerical value by determining whether workout trajectories match each other, so as to determine the early regularity, and regularity may be determined by using any one of various methods, for example, based on a distance between workout trajectories or a length of time series.

The objectification index calculator 422 may determine that the first user has high regularity of the 12 reps 711 through 714, 721 through 724, and 731 through 734, the second user has low reps 814, 821, 822, and 823, i.e., low middle regularity, and the third user has low reps 932 through 934, i.e., low latter regularity, and assign scores accordingly.

The objectification index calculator 422 may also determine an ascent starting point standard deviation, a descent starting point standard deviation, a height standard deviation, an ascending section velocity standard deviation, and a descending section velocity standard deviation of the workout trajectories of the 12 reps for each user, and assign scores according to a pre-set standard.

Also, the objectification index calculator 422 may calculate the individual objectification index by further referring to a performance time during which all reps configuring one set has been performed.

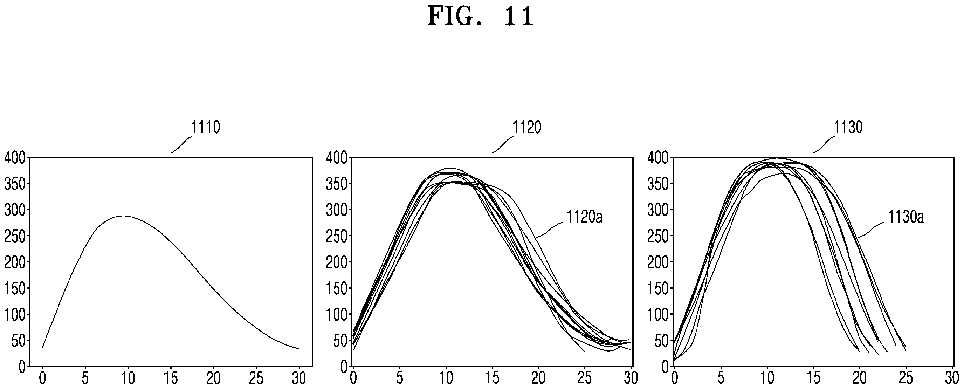

FIG. 11 illustrates an example of detecting workout trajectories of reps of a first user 1120 and a second user 1130 who have a same $PMW_{estimation}$ value for a chest press machine, according to an embodiment. In FIG. 11, an x-axis denotes time and a y-axis denotes movement displacement.

The first user 1120 and the second user 1130 may initially receive a same workout program, based on same $PMW_{estimation}$. A workout program for the chest press machine may include an initial target weight, reps, a moving velocity guide, and a respiration guide of fitness equipment appropriate to a user to work out in an optimum workout trajectory 1110.

Referring to FIG. 11, it is determined that movement displacement of the first user 1120 is higher than the optimum workout trajectory 1110, but an ascent starting point, a descent starting point, an ascending section average velocity, and a descending section average velocity are similar to the optimum workout trajectory 1110. Also, it is identified that a deviation 1120a of 11 workout trajectories is small.

For the second user 1130, it is identified that movement displacement is high, and a descent starting point and a descending section average velocity are too fast. Also, it is identified that a deviation 1130a of 11 workout trajectories is large.

A PMW guider may increase a $PMW_{individual}$ value of the first user 1120, and decrease a $PMW_{individual}$ value of the second user 1130.

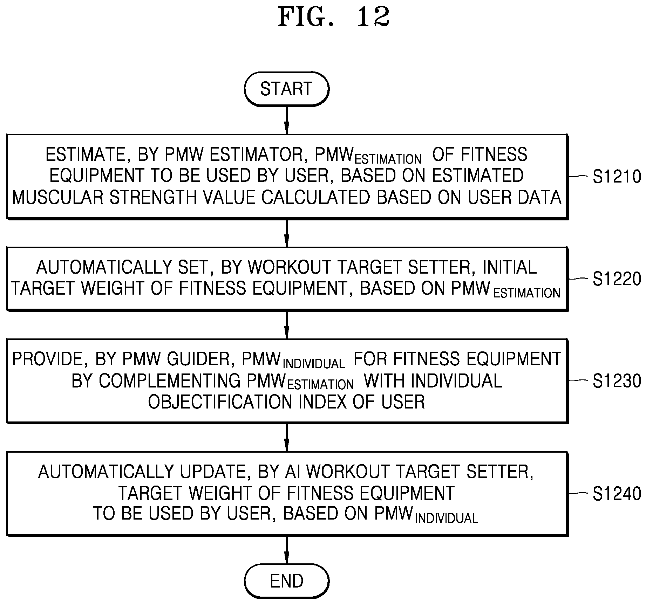

FIG. 12 is a flowchart of an AI workout guide method according to an embodiment.

A PMW estimator estimates $PMW_{estimation}$ of fitness equipment to be used by a user, based on an estimated muscular strength value calculated based on user data (operation S1210). A workout target setter automatically sets an initial target weight of the fitness equipment, based on $PMW_{estimation}$ (operation S1220).

A PMW guider provides $PMW_{individual}$ for the fitness equipment by complementing $PMW_{estimation}$ with an individual objectification index of the user, including athletic performance obtained based on the initial target weight for a certain period of time (operation S1230). An AI workout target setter automatically updates a target weight of the fitness equipment, based on $PMW_{individual}$ (operation S1240).

According to an embodiment, an AI workout guide apparatus may automatically set a target weight appropriate to a user for each piece of fitness equipment when user data including at least some of a gender, age, weight, height, BMI, and body fat percentage is input.

According to an embodiment, an AI workout guide apparatus learns and analyzes athletic performance of a user and continuously updates a target weight of fitness equipment used by the user, thereby enabling the user to use the fitness equipment in an optimum manner.

According to an embodiment, an AI workout guide apparatus is newly offered a method of using fitness equipment according to athletic performance of a user and a changed physical condition of the user, thereby increasing a workout effect.

According to an embodiment, an AI workout guide apparatus may automatically set a target weight appropriate to a user for each piece of fitness equipment, according to a purpose of workout of the user.

Methods according to embodiments may be recorded on a computer-readable recording medium by being implemented in a form of program commands executed by using various computers. The computer-readable recording medium may include at least one of a program command, a data file, or a data structure. The program commands recorded in the computer-readable recording medium may be specially designed or well known to one of ordinary skill in the computer software field.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

**Claims**

1. An artificial intelligence (AI) workout guide apparatus comprising:

   a personal maximum weight (PMW) estimator configured to estimate $PMW_{estimation}$ of fitness equipment to be used by a user, based on an estimated muscular strength value calculated based on user data; and
   a PMW guider configured to provide $PMW_{individual}$ for the fitness equipment by complementing the $PMW_{estimation}$ with an individual objectification index of the user,
   wherein PMW indicates muscular strength exerted by an individual against a resistance of a weight with an utmost effort, and the individual objectification index comprises at least some of a weight of the fitness equipment, a number of repetitions (reps), a number of sets, a workout trajectory, a moving velocity, and regularity of reps in units of sets, which are determined when the user uses the fitness equipment for a pre-set period of time.

2. The AI workout guide apparatus of claim 1, further comprising a workout target setter configured to automatically set an initial target weight of the fitness equipment to be used by the user, based on the $PMW_{estimation}$.

3. The AI workout guide apparatus of claim 1, further comprising an AI workout target setter configured to automatically update a target weight of the fitness equipment to be used by the user, based on the $PMW_{individual}$.

4. The AI workout guide apparatus of claim 1, wherein the PMW guider comprises a PMW updater configured to update the $PMW_{individual}$ to a value greater than the $PMW_{estimation}$ when the individual objectification index is equal to or greater than a first reference value, and update the $PMW_{individual}$ to a value less than $PMW_{estimation}$ when the individual objectification index is equal to or less than a second reference value.

5. The AI workout guide apparatus of claim 4, wherein the PMW updater is further configured to newly update the updated $PMW_{individual}$ based on the individual objectification index in units of the pre-set periods of time.

6. The AI workout guide apparatus of claim 1, wherein the PMW guider comprises a display configured to display a target weight of the fitness equipment, the target weight being automatically updated based on the $PMW_{individual}$.

7. The AI workout guide apparatus of claim 1, wherein the PMW guider is further configured to, when the workout trajectory is used as the individual objectification index, determine completion of the workout trajectory by using an ascent starting point, a descent starting point, an ascending section average velocity, a descending section average velocity, and a height, which are determined from the workout trajectory, convert a degree of the completion of the workout trajectory into a numerical value, and use the numerical value as the individual objectification index.

8. The AI workout guide apparatus of claim 1, wherein the PMW guider is further configured to, when the reps are used as the individual objectification index, determine completion of the reps based on regularity between workout trajectories of all reps configuring one set and a performance time of performing the all reps configuring the one set, convert a degree of the completion of the reps into a numerical value, and use the numerical value as the individual objectification index.

9. An artificial intelligence (AI) workout guide method comprising:

estimating, by a PMW estimator, $PMW_{estimation}$ of fitness equipment to be used by a user, based on an estimated muscular strength value calculated based on user data; and
providing, by a PMW guider, $PMW_{individual}$ for the fitness equipment by complementing the $PMW_{estimation}$ with an individual objectification index of the user,
wherein the individual objectification index comprises at least some of a weight of the fitness equipment, a number of repetitions (reps), a number of sets, a workout trajectory, a moving velocity, and regularity of reps in units of sets, which are determined when the user uses the fitness equipment for a pre-set period of time.

10. The AI workout guide method of claim 9, further comprising automatically setting, by a workout target setter, an initial target weight of the fitness equipment, based on the $PMW_{estimation}$.

11. The AI workout guide method of claim 9, further comprising automatically updating, by an AI workout target setter, a target weight of the fitness equipment, based on the $PMW_{individual}$.

12. The AI workout guide method of claim 9, wherein the providing of the $PMW_{individual}$ comprises updating, by a PMW updater, the $PMW_{individual}$ to a value greater than the $PMW_{estimation}$ when the individual objectification index is equal to or greater than a first reference value, and the $PMW_{individual}$ to a value less than $PMW_{estimation}$ when the individual objectification index is equal to or less than a second reference value.

13. The AI workout guide method of claim 12, wherein the providing of the $PMW_{individual}$ comprises newly updating the updated $PMW_{individual}$ in units of the pre-set periods of time, based on the individual objectification index.

14. The AI workout guide method of claim 9, wherein the providing of the $PMW_{individual}$ comprises displaying, through a display, the $PMW_{individual}$ or a change amount of maximum weight that is liftable when the fitness equipment is used.

15. The AI workout guide method of claim 9, wherein, when the workout trajectory is used as the individual objectification index, completion of the workout trajectory is determined by using an ascent starting point, a descent starting point, an ascending section average velocity, a descending section average velocity, and a height, which are determined from the workout trajectory, a degree of the completion of the workout trajectory is converted into a numerical value, and the numerical value is used as the individual objectification index.

**16.** The AI workout guide method of claim 9, wherein, when the reps are used as the individual objectification index, completion of the reps is determined based on regularity between workout trajectories of all reps configuring one set, a degree of the completion of the reps is converted into a numerical value, and the numerical value is used as the individual objectification index.

**17.** A computer-readable recording medium storing commands for enabling a computing device to:

estimate, by a PMW estimator, $PMW_{estimation}$ of fitness equipment to be used by a user, based on an estimated muscular strength value calculated based on user data; and

provide, by a PMW guider, $PMW_{individual}$ for the fitness equipment by complementing the $PMW_{estimation}$ with an individual objectification index of the user,

wherein the individual objectification index comprises at least some of a weight of the fitness equipment, a number of repetitions (reps), a number of sets, a workout trajectory, a moving velocity, and regularity of reps in units of sets, which are determined when the user uses the fitness equipment for a pre-set period of time.

# FIG. 1

FIG. 2

# FIG. 3

# FIG. 4

400

410 **PMW ESTIMATOR**

412 **MUSCULAR STRENGTH ESTIMATOR**

414 **DETECTOR**

USER DATA

420 **PMW GUIDER**

422 **OBJECTIFICATION INDEX CALCULATOR**

424 **PMW UPDATER**

430 **WORKOUT TARGET SETTER**

440 **AI WORKOUT TARGET SETTER**

450 **DISPLAY**

# FIG. 5

540 — SHOULDER PRESS MACHINE

550 — **25 kg** | VG1

V1

530

520

ROM

541 — ⊙ Free
542 — ⦿ Standard
543 — ⊙ MuscleBuilding
544 — ⊙ Conditioning

A
B

510

500

DRAX

0

0 set

NEXT SET

RESET

# FIG. 6

H 612

1.0

0.8

0.6 — V1 — S610    V2 — S620

0.4

0.2

0    1    2    3    4    5

t1   t2         t3   t4

611                613

# FIG. 7

EP 4 207 212 A1

FIG. 8

EP 4 207 212 A1

# FIG. 9

# FIG. 10

EP 4 207 212 A1

# FIG. 11

# FIG. 12

START

ESTIMATE, BY PMW ESTIMATOR, $PMW_{ESTIMATION}$ OF FITNESS EQUIPMENT TO BE USED BY USER, BASED ON ESTIMATED MUSCULAR STRENGTH VALUE CALCULATED BASED ON USER DATA ⌐ S1210

AUTOMATICALLY SET, BY WORKOUT TARGET SETTER, INITIAL TARGET WEIGHT OF FITNESS EQUIPMENT, BASED ON $PMW_{ESTIMATION}$ ⌐ S1220

PROVIDE, BY PMW GUIDER, $PMW_{INDIVIDUAL}$ FOR FITNESS EQUIPMENT BY COMPLEMENTING $PMW_{ESTIMATION}$ WITH INDIVIDUAL OBJECTIFICATION INDEX OF USER ⌐ S1230

AUTOMATICALLY UPDATE, BY AI WORKOUT TARGET SETTER, TARGET WEIGHT OF FITNESS EQUIPMENT TO BE USED BY USER, BASED ON $PMW_{INDIVIDUAL}$ ⌐ S1240

END

Europäisches
Patentamt

European
Patent Office

Office européen
des brevets

# EUROPEAN SEARCH REPORT

Application Number

**EP 22 21 4919**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/001181 A1 (VON PRELLWITZ LEONARDO [US] ET AL) 4 January 2018 (2018-01-04) * paragraphs [0002], [0004], [0032], [0033] – [0039], [0047], [0048], [0051], [0052], [0058], [0062], [0065], [0073], [0082], [0088] – [0090], [0094]; figures 4,8 * ----- | 1-17 | INV. G16H20/30 A63B24/00 |
| X | KR 2003 0068790 A (KIM JONG GWAN [KR]) 25 August 2003 (2003-08-25) * the whole document * ----- | 1-17 | |
| A | Hristo Novatchkov ET AL: "Artificial intelligence in sports on the example of weight training", Journal of sports science & medicine, 1 March 2013 (2013-03-01), pages 27-37, XP055431798, Retrieved from the Internet: URL:https://www.ncbi.nlm.nih.gov/pmc/articles/PMC3761781/pdf/jssm-12-27.pdf [retrieved on 2023-04-19] * the whole document * ----- | 1-17 | |
| | | | **TECHNICAL FIELDS SEARCHED (IPC)** G16H A63B |

The present search report has been drawn up for all claims

| Place of search | Date of completion of the search | Examiner |
|---|---|---|
| Munich | 21 April 2023 | Hendrikse, Natalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 4919

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2023

| Patent document cited in search report | Publication date | Patent family member(s) | Publication date |
|---|---|---|---|
| US 2018001181 A1 | 04-01-2018 | US 2018001181 A1<br>US 2022249937 A1 | 04-01-2018<br>11-08-2022 |
| KR 20030068790 A | 25-08-2003 | NONE | |

EPO FORM P0459

For more details about this annex : see Official Journal of the European Patent Office, No. 12/82

**EP 4 207 212 A1**

**Patent documents cited in the description**

- KR 1020210190376

- KR 1020220152001